# FASCICULE DE BREVET EUROPEEN

(11) **EP 0 355 154 B1**
(45) Date de publication et mention de la délivrance du brevet: **09.11.1994**
(21) Numéro de dépôt: 89902523.3
(22) Date de dépôt: 09.02.1989
(51) Int. Cl.: A45D 40/00

(54) **PROCEDE ET DISPOSITIFS DE MAQUILLAGE PAR TRANSFERT DE PIGMENTS COLORES SUR LA PEAU A PARTIR D'UN SUPPORT SOUPLE PREDECOUPE**
VERFAHREN UND VORRICHTUNGEN ZUM SCHMINKEN DURCH ÜBERTRAGUNG VON FARBIGEN PIGMENTEN AUF DIE HAUT MITTELS EINER BIEGSAMEN VORGESCHNEIDETEN UNTERLAGE
PROCESS AND DEVICES FOR MAKE UP BY TRANSFERRING COLOURED PIGMENTS TO THE SKIN FROM A CUT-OUT SOFT SUPPORT

(30) Priorité: 10.02.1988 FR 8801666
(43) Date de publication de la demande: 28.02.1990
(73) Titulaire: CARBONNIER, Isabelle, F-51100 Reims (FR)
(72) Inventeur: CARBONNIER, Isabelle, F-51100 Reims (FR)
(86) Numéro de dépôt international: FR8900051
(87) Numéro de publication internationale: WO8907407

(56) Documents cités:
- EP-A- 0 252 001
- FR-A- 825 935
- FR-A- 941 489
- US-A- 3 308 837
- US-A- 3 568 684

## Description

Les moyens traditionnels d'application de fards sur la peau se limitent aux pinceaux et aux tampons combinés aux bouchons des récipients de fard.

Pour en faciliter l'application par des personnes malhabiles ou peu douées de sens esthétique, on a proposé, dans les brevets FR A 941 489 et FR A 1 577 258, de guider le crayon ou autre moyen d'application du fard au moyen d'un cache convenablement découpé servant de pochoir. Les procédés au pochoir délimitent correctement les surfaces de peau à enduire, mais obligent l'usager à recourir aux moyens traidtionnels d'application, lui laissant le choix de couleurs et de leur agencement.

Un procédé de décalcomanie est par ailleurs décrit, dans son application aux paupières, dans les brevets FR A 941 489 et US 3 568 684 : une feuille de papier recouverte d'une couche de colle lavable à l'eau reçoit par impression une pellicule qui porte un motif coloré bien délimité à transférer sur la peau. On humecte pour diminuer l'adhérence entre la pellicule et le papier et on enlève la couche protectrice qui recouvre normalement ladite pellicule, laquelle est alors appliquée sur la paupière. Il ne s'agit pas à proprement parler d'un maquillage.

Les procédés de décalcomanie ne permettent pas de faire coïncider avec précision le contour des plages colorées avec celui de la paupière ou autre portion de peau a farder. En outre, la colle adhère à la peau et doit être enlevée en évitant de détériorer le motif, ce qui est délicat et requiert que la peinture utilisée ait une composition spéciale résistant au lavage : il ne s'agit donc pas de pigments traditionnels de maquillage.

Le brevet EP 0 252 001 décrit quant à lui un conditionnement pour doses unitaires de produits divers, notamment pour échantillons de poudres cosmétiques. Toutefois, ce brevet prévoit des dispositions incompatibles avec le principe d'un transfert direct des produits sur la peau.

L'invention supprime les inconvénients précédemment évoqués au moyen d'un procédé de maquillage par transfert de matières colorées sur la peau à partir d'un support prédécoupé relativement souple appliqué sur la peau et enlevé après transfert.

Selon l'invention, ce procédé est caractérisé en ce que des pigments ou laques colorés pulvérulents de type cosmétique sont déposés sur ledit support pour constituer une zone pigmentée ayant le contour d'une région prédéterminée de la peau, avec une répartition des différentes matières colorées et de leur densité dans ladite zone propres à l'obtention de l'effet de maquillage final, l'adhérence des pigments entre eux et au support étant, dans la couche superficielle, plus faible que leur adhérence à la peau de façon que leur transfert sur la peau se fasse par simple application du support sur la portion de peau à farder.

L'invention concerne aussi un ensemble de maquillage et un dispositif de maquillage selon les revendications 3 et 11

D'autres caractéristiques ainsi que les avantages de l'invention apparaîtront clairement à la lecture de la description ci-après.

Aux dessins annexés :
La figure 1 représente un dispositif de maquillage conforme à l'invention, appliqué autour du contour d'un oeil droit ouvert ;
La figure 2 représente schématiquement un dispositif de maquillage destiné à la paupière gauche ;
La figure 3 illustre un mode de réalisation d'une zone pigmentée dans laquelle les pigments sont répartis sur le support selon une pluralité de plages de dépôt qui se recouvrent partiellement ;
Les figures 4 à 6 représentent les différentes matrices utilisées successivement pour réaliser les plages de dépôt illustrées figure 3 ;
La figure 7 est un organigramme des opérations de fabrication du dispositif ;
La figure 8 représente un exemple de chaîne de fabrication ;
Les figures 9 à 11 représentent des modes d'exécution du dispositif d'alimentation en pigments ;
Les figures 12 et 13 représentent deux modes d'exécution du dispositif de pressage ; et
Les figures 14 à 16 représentent des modes d'exécution du dispositif de découpe.

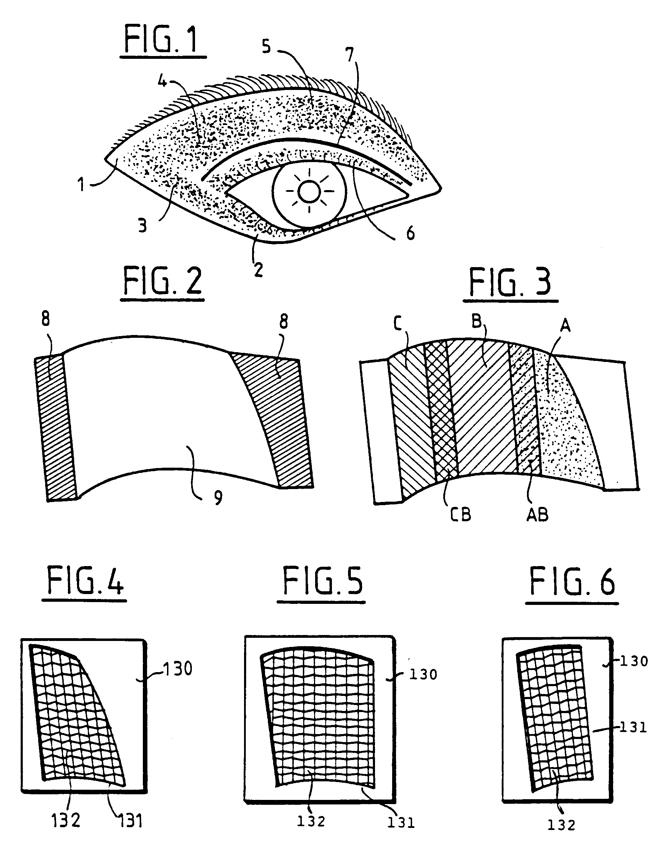

A la figure 1, on voit que le support souple 1 est prédécoupé pour s'appliquer exactement autour de l'oeil droit ouvert et est recouvert de pigments, en des régions 2 à 6, de couleurs différentes choisies en fonction de la couleur des yeux et du teint du visage.

Une concentration de pigments suivant une ligne 7 donne l'impression d'un trait de couleur analogue à celui obtenu traditionnellement à l'aide d'un crayon spécial.

A la figure 2, on a représenté un support souple prédécoupé pour s'appliquer exactement sur la paupière de l'oeil gauche fermé, et qui comporte une zone non pigmentée 8 et une zone pigmentée 9. La figure 3 montre que cette dernière se décompose elle-même en plages de dépôt de couleurs différentes A, B et C avec des recouvrements partiels AB et CB.

Le support est avantageusement en non-tissé, par exemple constitué de fibres de viscose liées chimiquement par des latex. Un tel produit est facile à découper et possède une souplesse, variable selon les types mais suffisante pour épouser, lors de l'application, la forme de la région à maquiller. Il s'agit de préférence d'un produit absorbant, mais dont une face est rendue non absorbante par un film de polyéthylène. Il a une bonne résistance à la rupture, ne se froisse pas et n'est pas excessivement déformable, ce qui entraînerait une répartition irrégulière du pigment sur la peau, est agréable au toucher et est résistant au liant.

Les pigments ou laques sont retenus sur le support de façon stable en présence de secousses ou de choc, par une colle non toxique pour la peau ou l'oeil. Il s'agit d'une colle redéposable, appliquée sur le support puis séchée avant de recevoir les pigments ou les laques. Elle permet un bon relarguage des pigments qu'elle adsorbe sans les empêcher de se redéposer.

La colle est absorbée par le support, mais ne le traverse pas grâce au film de polyéthylène ; contrairement à une colle classique, elle ne diffuse pas à travers les différentes strates de pigment ou de laques.

Les pigments ou laques sont des matières colorées solides couramment utilisées en cosmétique.

De façon connue en soi, on incorpore aux substances colorantes monochromes des liants, tels que huiles minérales ou végétales, esters gras et produits hydrophiles de types polyols, ces derniers faciliant le démaquillage à l'eau.

Chaque colorant monochrome est dispersé dans un liant approprié à l'aide d'une turbine rapide et l'on obtient des couleurs complexes par simple mixage de plusieurs des poudres monochromes ainsi obtenues. On homogénéise le mélange au moyen d'une turbine rapide, et on y ajoute en même temps un fixateur, un plastifiant, des conservateurs et des parfums, de façon connue en soi.

Le fixateur, avantageusement la polyvinylpyrrolidone, hydrosoluble, utilisée avec une proportion inférieure à 1 % dans le produit fini pour en maintenir la souplesse, et qui a en outre la propriété de lier les pigments, a surtout pour fonction d'assurer une protection de la surface du maquillage contre un contact manuel. Il est soluble dans la crème applicatrice dont sera avantageusement enduite la peau avant l'application du dispositif, pour assurer une meilleure adhérence des pigments ou des laques.

L'agent plastifiant, nécessaire pour maintenir la souplesse du produit lors de la manipulation, et utilisé à la concentration de 0,1 % environ, peut être de différents types connus.

La crème applicatrice, qui présente un très bon pouvoir solvant vis-à-vis des liants associés aux pigments (effet dispersant des pigments), rendant ainsi aisé l'emploi du produit sans que l'usager n'ait à appliquer de trop fortes pressions, contient à cet effet une huile de paraffine ou de ricin, additionnée d'huiles végétales qui lui confèrent un bon toucher cosmétique, et de 2 à 10 % d'émulgateurs non ioniques (cette proportion donnant le minimum de toxicité pour l'oeil).

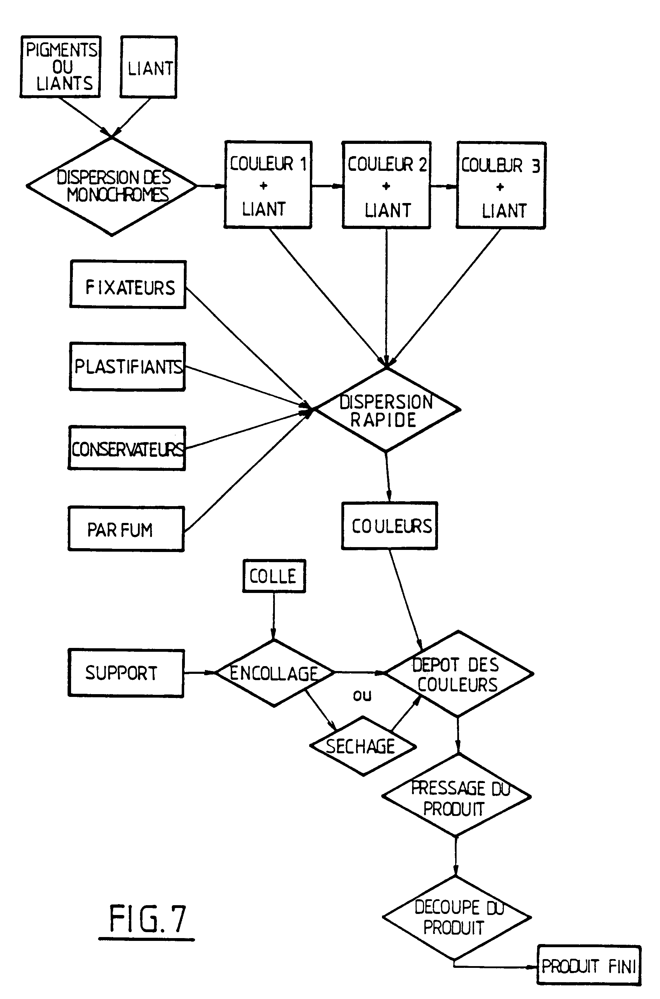

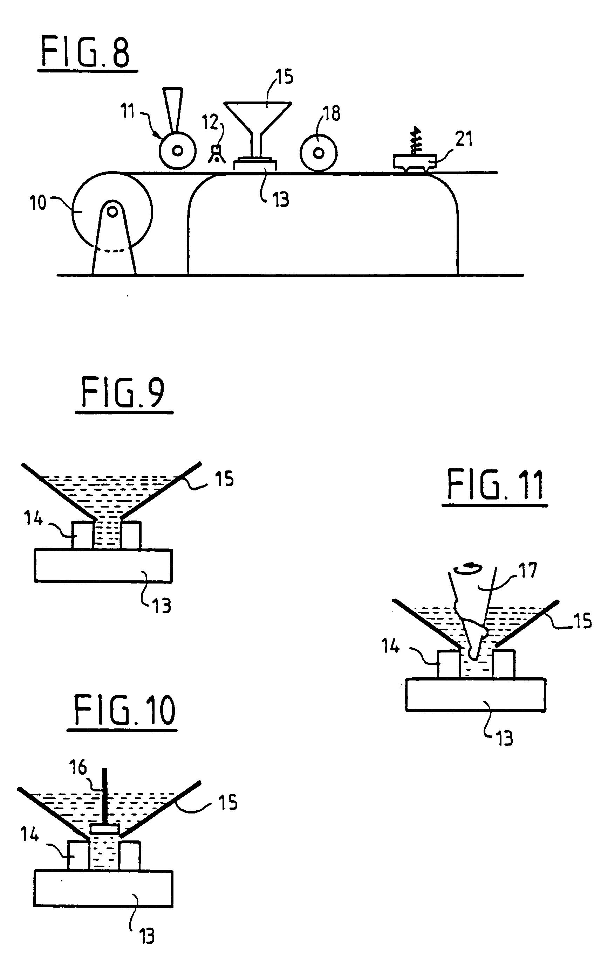

Le procédé de fabrication est illustré par la figure 7 et la chaîne par la figure 8.

Une bande de support défile de façon continue à partir d'un rouleau 10.

L'encollage du support se réalise par un simple rouleau 11 du type encreur.

Un séchage peut être effectué extemporanément par passage de la bande dans un tunnel 12 à 40°C ou par un courant d'air chaud.

La forme de chaque plage de dépôt de couleur (A, B, C) est définie par une matrice spécifique 13, telle que celles représentées sur les figures 4 à 6 qui comprennent chacune un corps 130 muni d'une découpe 131 présentant la forme correspondante et recouverte d'une grille 132 au travers de laquelle peuvent passer les pigments ou les laques, avec une répartition homogène.

Le dispositif d'alimentation 14 des matrices 13 (figure 8), plus particulièrement représentées figures 4 à 6, permet de déposer les pigments en quantité fixée. Il comprend un sabot d'alimentation 14 (figures 9 à 11) associé à un réservoir de pigments 15.

Un piston de bourrage 16 (figure 10) ou une vis sans fin 17 (figure 11) peuvent pousser la couleur vers la grille.

Comme le montre la figure 3, on peut utiliser successivement les matrices représentées figures 4 à 6 pour obtenir la zone pigmentée souhaitée dans laquelle les couleurs sont juxtaposées, avec recouvrements partiels possibles AB, CB des plages A, B, C ; pour obtenir un dégradé de couleurs, il suffit alors de passer un pinceau sur la région fardée.

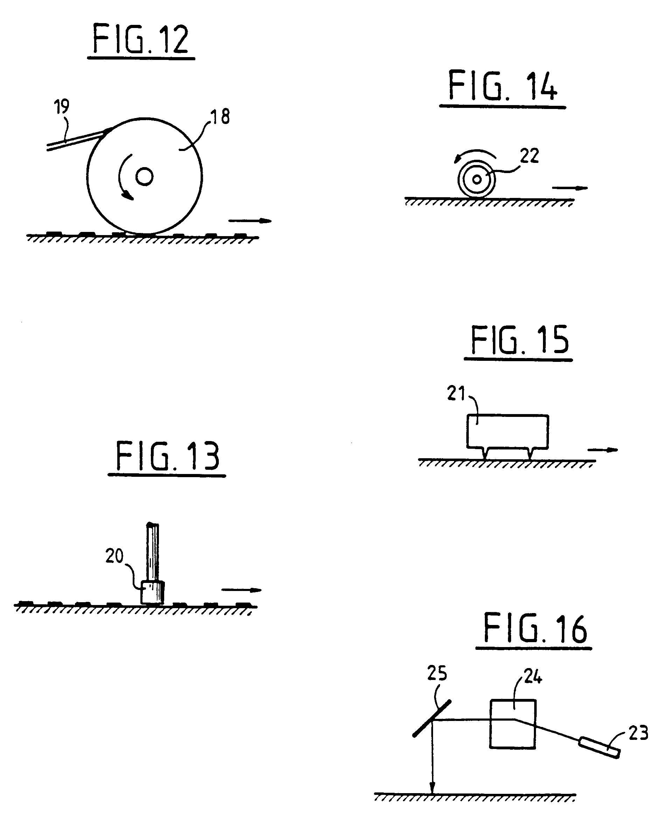

La couche de colorant est homogénéisée en épaisseur au moyen d'un rouleau presseur 18 (figures 8 et 12) qui effectue un compactage du pigment sur la bande. Un dispositif de nettoyage 19 est associé au rouleau.

En variante, on peut utiliser un piston alternatif 20 (figure 13).

La découpe du produit s'effectue, soit au moyen d'un emporte-pièce à la forme 21 (figures 8 et 15), soit en continu au moyen de couteaux circulaires 22 (figure 14), soit à l'aide d'un faisceau laser obtenu à l'aide d'un générateur 23 (figure 16) associé à des miroirs 24, 25.

La couche de colorant compactée comporte plusieurs strates dont celle qui vient au contact de la peau est transférée sur celle-ci. La strate intérieure la plus proche de la surface du support n'est pas transférée et reste collée au support : il n'y a donc aucun contact de la colle avec la peau.

Les zones non couvertes de pigment 8 (figure 2) facilitent la mise en place précise du dispositif sur la région à farder. L'application est facile et immédiate et l'enlèvement du support après dépôt se fait aisément sans détériorer le maquillage. L'effet esthétique est entièrement déterminé par la sélection des pigments et l'arrangement des couleurs effectués à la fabrication.

Un ensemble de maquillage pourra comporter un jeu de supports prédécoupés pour s'adapter à différentes régions à maquiller : pommettes, tempes, yeux, paupières, et autres, en tenant compte des morphologies différentes des visages, et pour chaque région, répondre à des gammes de caractéristiques colorées différentes en fonction des personnes, des effets de maquillage recherchés et de la mode.

## Revendications

1. Procédé de maquillage par transfert de matières colorées sur la peau à partir d'un support prédécoupé relativement souple appliqué sur la peau et enlevé après transfert,
caractérisé en ce que des pigments ou laques colorés pulvérulents de type cosmétique sont déposés sur ledit support (1) pour constituer une zone pigmentée (9) ayant le contour d'une région prédéterminée de la peau, avec une répartition des différentes matières colorées et de leur densité dans ladite zone propres à l'obtention de l'effet de maquillage final, l'adhérence des pigments entre eux et au support étant, dans la couche superficielle, plus faible que leur adhérence à la peau de façon que leur transfert sur la peau se fasse par simple application du support sur la portion de peau à farder.

2. Procédé selon la revendication 1,
caractérisé par l'application préalable sur la peau d'une crème ayant un fort pouvoir solvant vis-à-vis des liants associés aux pigments.

3. Ensemble de maquillage,
caractérisé en ce qu'il comprend un jeu de supports prédécoupés pour s'adapter à différentes régions à maquiller, à différents effets de maquillage et à des personnes différentes, chaque support étant au moins partiellement recouvert par des pigments ou laques colorés pulvérulents de type cosmétique disposés de manière à constituer une zone pigmentée (9) ayant le contour d'une région prédéterminée de la peau, avec une répartition des différentes matières colorées et de leur densité dans ladite zone propres à l'obtention de l'effet de maquillage final, l'adhérence des pigments, entre eux et dans la couche superficielle, étant plus faible que leur adhérence à la peau, de façon que leur transfert sur la peau se fasse par simple application du support sur la portion de peau à farder.

4. Procédé selon la revendication 1,
caractérisé en ce que le support est constitué par une feuille en non-tissé.

5. Procédé selon la revendication 4,
caractérisé en ce que le non-tissé est constitué de fibres de viscose liées chimiquement par des latex.

6. Procédé selon les revendications 4 ou 5,
caractérisé en ce que le non-tissé est absorbant et que la feuille est revêtue sur une face d'un film de polyéthylène.

7. Procédé selon la revendication 1,
cvaractérisé par un encollage du support avant le dépot des pigments, au moyen d'une colle liquide redéposable.

8. Procédé selon la revendication 1,
caractérisé par la dispersion de chacun des constituants colorés monochrome de chaque matière colorée dans un liant approprié avant le mélange desdits constituants.

9. Procédé selon la revendication 1,
caractérisé par le dépôt, en quantité prédéterminée, de chaque matière colorée dans une plage de dépôt prédéterminée du support, suivi du compactage par pressage.

10. Procédé selon la revendication 9,
caractérisé par la juxtaposition desdites plages de dépôt avec chevauchement partiel sur le support.

11. Dispositif de maquillage,
caractérisé en ce qu'il comprend un support au moins partiellement recouvert par des pigments ou laques colorés pulvérulents de type cosmétique disposés de manière à constituer une zone pigmentée (9) ayant le contour d'une région prédéterminée de la peau, avec une répartition des différentes matières colorées et de leur densité dans ladite zone propres à l'obtention de l'effet de maquillage final, l'adhérence des pigments, entre eux et dans la couche superficielle, étant plus faible que leur adhérence à la peau, de façon que leur transfert sur la peau se fasse par simple application du support sur la portion de peau à farder.

12. Dispositif selon la revendication 11,
caractérisé en ce que ledit support comporte, en plus d'une zone pigmentée, des zones non pigmentées destinées à faciliter sa mise en coïncidence avec ladite région.

## Claims

1. A make up device by transferring coloured materials on to the skin from a relatively flexible support applied to the skin and removed after transfert,
said method comprising the steps of depositing on said support (1) powdery coloured pigments or lacquers of the coosmetic type bonded together by means of binder to form a pigmented layer (9) having the contour of a predetermined region of the skin, with a distribution of the respective powdery coloured pigments or lacquers and of their respective densities in said zone so as to obtain the final make-up effect, said layer having an outer stratum in which the adherence of the pigments or lacquers with each other and with the support is weaker than their adherence to the skin and transferring said pigmented layer to the skin by application of at least said outer stratum of the support provided with the pigmented layer on the skin portion to be make-up.

2. A method according to claim 1,
further comprising the prior step of applying on the skin a cream having a hight solvent power with respect to said binders.

3. A make-up device,
comprising a set of pre-cut supports adapted to match the surface of different portions of a person's skin, in any of a pluraly of arbitrary designs and patterns of colour, each of said supports having deposited thereon a pigmented layer of powdery coloured pigments or lacquers of the cosmetic type bonded together by means of binders to form a pigmented layer (9) having the contour of a predetermined region of the skin, with a distribution of the respective powdery coloured pigments or lacquers and of their respective densities in said zone so as to obtain the final make-up effect, said layer having an outer stratum in which the adherence of the pigments or lacquers wich each other and with the support is weaker than their adherence to the skin and transferring said pigmented layer to the skin by application of at least said outer stratum of the skin portion to be make-up.

4. Method according to claim 1,
wherein said support is formed by a foil of non woven fabric.

5. Method according to claim 4,
wherein the non woven fabric is formed of viscose fibres bonded chemically by means of latexes.

6. Method according to clain 4 or 5,
wherein the non woven fabric is absorbent and the foil is coated on one side with a polyethylene film.

7. Method according to claim 1,
wherein a further comprising the step of coating the support with an adhesive before depositing the pigments or lacquers said adhesive being a liquid adhesive adapted to the redeposited.

8. Method according to claim 1,
wherein the said pigments or lacquers are prepared by dispersing in said binders respective monochrome coloured constituents of each pigments or lacquers and subsequently mixing said constituents.

9. Method according to claim 1,
wherein the step of depositing the pigments or lacquers comprises the deposition, in predetermined amounts, of each coloured constituant of said pigments or lacquers in a predetermined deposition zone of the support and subsequently compacting the coloured constituants by pressing.

10. Method according to claim 9,
wherein the respective deposition zones are partially overlapping on the support.

11. A make-up device,
wherein said support comprises partially pigmented zones on wich powdrey coloured pigments or lacquers of the cosmetic type bonded together by means of binders to form a pigmented layer (9) having the contour of a predetermined region of the skin, the distribution of the respective powdery coloured pigments or lacquers and of their respective densities in said zone required for obtaining the final make-up effect, said layer having an outer stratum in wich the adherence of the pigments or lacquers with each other and with the support being weaker than their adherence to the skin so that tranfer thereof to the skin takes place by simply applying the support on the skin portion to be make-up.

12. Method according to claim 11,
wherein said support futher comprises non pigmented zones designed for facilitating the positioning of said support on a predetermined region of the person's skin.

## Patentansprüche

1. Schminkverfahren durch Übertragung von Farbstoffen auf die Haut mittels einer vorgestanzten, relativ geschmeidigen Auflage, die auf die Haut gedrückt, und nach der Übertragung wieder abgenommen wird,
gekennzeichnet dadurch, dass gefärbte, pulverige Pigmente oder Lacke kosmetischer Art auf die besagte Auflage (1) deponiert werden, um eine Pigmentzone (9) zu schaffen, die die Kontur einer festgelegten Partie der Haut hat; die Verteilung der verschiedenen Farbstoffe und ihres Dichtigkeitsgrades auf der besagten Zone ist so, dass der Effekt einer fertiggeschminkten Hautpartie erreicht wird, wobei die Adhäsion der Pigmente untereinander und an die Auflage in der Oberflächenschicht schwächer ist als ihre Adhäsion an die Haut, so dass ihre Übertragung auf die Haut durch einfaches Andrücken der Auflage auf die zu schminkende Hautpartie geschieht.

2. Verfahren nach Anspruch 1,
gekennzeichnet durch ein vorhergehendes Auftragen auf die Haut einer Creme mit starker Lösungsfähigkeit gegenüber den mit den Pigmenten verbundenen Bindemitteln.

3. Schmink-Ensemble,
gekennzeichnet dadurch, dass es einen Satz mit Auflagen enthält, die an verschiedene zu schminkende Hautpartien, verschiedene Schminkeffekte und verschiedene Personen adaptiert werden können, wobei jede Auflage mindestens teilweise bedeckt ist mit gefärbten, pulverigen Pigmenten oder Lacken kosmetischer Art, und zwar so, dass eine Pigmentzone (9) geshaffen wird, die die Kontur einer festgelegten Partie der Haut hat; die Verteilung der verschiedenen Farbstoffe und ihres Dichtigkeitsgrades auf der besagten Zone ist so, dass der Effekt einer fertiggeschminkten Hautpartie erreicht wird, wobei die Adhäsion der Pigmente untereinander und an die Auflage in der Oberflächenschicht schwächer ist als ihre Adhäsion an die Haut, so dass ihre Übertragung auf die Haut durch einfaches Andrücken der Auflage auf die zu schmink-Hautpartie geschieht.

4. Verfahren nach Anspruch 1,
gekennzeichnet dadurch, dass die Auflage aus Faservlies besteht.

5. Verfahren nach Anspruch 4,
gekennzeichnet dadurch, dass das Faservlies aus chemisch mit Latex verbundenen Viskosefasern besteht.

6. Verfahren nach Anspruch 4oder 5,
gekennzeichnet dadurch, dass das Faservlies absorbierend und auf einer Seite mit einem Polyethylen-Film überzogen ist.

7. Verfahren nach Anspruch 1,
gekennzeichnet durch die mit flüssigem, wiederverwendbarem Leim vorgenommene Leimung der Auflage vor dem Auftragen der Pigmente.

8. Verfahren nach Anspruch 1, gekennzeichnet durch die Dispersion jedes der monochromen Farbbestandteile jedes Farbstoffs in einem entsprechenden Bindemittel vor der Mischung der besagten Bestandteile.

9. Verfahren nach Anspruch 1,
gekennzeichnet durch das Auftragen jedes - mengenmässig festgelegten - Farbstoffes in einen festgelegten Bereich der Auflage, mit anschliessendem Kompaktieren.

10. Verfahren nach Anspruch 9,
gekennzeichnet durch die Juxtaposition der genannten Bereiche mit teilweiser Überlappung über die Auflage.

11. Schminkvorrichtung,
gekennzeichnet dadurch, dass sie eine Auflage enthält, die mindestens teilweise bedeckt ist mit gefärbten, pulverigen Pigmenten oder Lacken kosmetischer Art, und zwar so, dass eine Pigmentzone (9) geschaffen wird, die die Kontur einer festgelegten Partie der Haut hat; die Verteilung der verschiedenen Farbstoffe und ihres Dichtigkeitsgrades auf der besagten Zone ist so, dass der Effekt einer fertiggeschminkten Hautpartie erreicht wird, wobei die Adhäsion der Pigmente untereinander und an die Auflage in ihrer Oberflächenschicht schwächer ist als ihre Adhäsion an die Haut, so dass ihre Übertragung auf die Haut durch einfaches Andrücken der Auflage auf die zu schminkende Hautpartie geschieht.

12. Vorrichtung nach Anspruch 11,
gekennzeichnet dadurch, dass die besagte Auflage ausser einer Pigmentzone auch nichtpigmentierte Zonen enthält, die die Deckungsgleichheit der Auflage mit der besagten Hautpartie erleichtern sollen.
